(19) Europäisches Patentamt
European Patent Office
Office européen des brevets

(11) Veröffentlichungsnummer: **0 155 634**
**B1**

(12) # EUROPÄISCHE PATENTSCHRIFT

(45) Veröffentlichungstag der Patentschrift: **13.06.90**

(51) Int. Cl.⁵: **C 07 D 291/06,** C 07 C 307/02

(21) Anmeldenummer: **85102885.2**

(22) Anmeldetag: **13.03.85**

(54) **Verfahren zur Herstellung von 6-Methyl-3,4-dihydro-1,2,3-oxathiazin-4-on-2,2-dioxid und dessen nicht-toxischen Salzen sowie der dabei als Zwischenprodukt(e) auftretenden Acetoacetamid-N-sulfonsäure(salze).**

(30) Priorität: **22.03.84 DE 3410439**

(43) Veröffentlichungstag der Anmeldung:
**25.09.85 Patentblatt 85/39**

(45) Bekanntmachung des Hinweises auf die Patenterteilung:
**13.06.90 Patentblatt 90/24**

(84) Benannte Vertragsstaaten:
**AT BE CH DE FR GB IT LI NL SE**

(56) Entgegenhaltungen:
**DE-A-2 453 063**
**FR-A-2 075 327**

(73) Patentinhaber: **HOECHST AKTIENGESELLSCHAFT**
**Postfach 80 03 20**
**D-6230 Frankfurt am Main 80 (DE)**

(72) Erfinder: **Clauss, Karl, Dr.**
**Im Birkenfeld 20**
**D-6233 Kelkheim (Taunus) (DE)**
Erfinder: **Linkies, Adolf, Dr.**
**Loreleistrasse 12**
**D-6230 Frankfurt am Main 80 (DE)**
Erfinder: **Reuschling, Dieter, Dr.**
**Beethovenstrasse 27**
**D-6308 Butzbach (DE)**

**EP 0 155 634 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

6-Methyl-3,4-dihydro-1,2,3-oxathiazin-4-on-2,2-dioxid ist die Verbindung der Formel

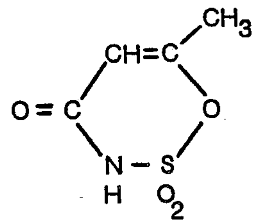

Infolge des aciden Wasserstoffs am Stickstoffatom ist die Verbindung zur Salzbildung (mit Basen) befähigt. Die nicht-toxischen Salze — wie z.B. das Na-, das K- und das Ca-Salz — können wegen ihres z.T. intensiven Süßgeschmacks als Sußstoffe auf dem Nahrungsmittelsektor verwendet werden, wobei das K-Salz ("Acesulfam K" oder auch nur "Acesulfam") vom besonderer Bedeutung ist.

Zur Herstellung des 6-Methyl-3,4-dihydro-1,2,3-oxathiazin-4-on-2,2-dioxids und dessen nicht toxischer Salze ist eine Reihe verschiedener Verfahren bekannt; vgl. Angewandte Chemie *85*, Heft 22 (1973) S. 965 bis 73, entsprechend International Edition Vol. 12, No. 11 (1973), S. 869—76. Praktisch alle Verfahren gehen von Chlor- oder Fluor-sulfonyliso-cyanat ($XSO_2NCO$ mit X = Cl oder F) aus. Das Chlor- bzw. Fluor-sulfonyliso-cyanat wird dann mit Monomethylacetylen, Aceton, Acetessigsäure, Acetessigsäure-tert.-butylester oder Benzylpropenylether (in einer meist mehrstufigen Reaktion) zu Acetoacetamid-N-sulfochlorid bzw. -fluorid umgesetzt, was unter der Einwirkung von Basen (wie z.B. methanolischer KOH) cyclisiert und die entsprechenden Salze des 6-Methyl-3,4-dihydro-1,2,3-oxa-thiazin-4-on-2,2-dioxids liefert. Aus den Salzen kann das freie Oxathiazinon gewünschtenfalls auf übliche Weise (mit Säuren) erhalten werden.

Ein weiteres Verfahren zur Herstellung der Oxathiazinon-Zwischenstufe Acetoacetamid-N-sulfofluorid geht aus von Amidosulfofluorid $H_2NSO_2F$, dem partiellen Hydrolyseprodukt des Fluorsulfonylisocyanats (DE—OS 2 453 063). Danach wird das Fluorid der Amidosulfonsäure $H_2NSO_2F$ mit einer etwa äquimolaren Menge des Acetoacetylierungsmittels Diketen in einem inerten organischen Lösungsmittel in Gegenwart eines Amins bei Temperatureen zwischen etwa −30 und 100°C umgesetzt; die Umsetzung verläuft nach folgender Reaktionsgleichung (mit Triethylamin als Amin):

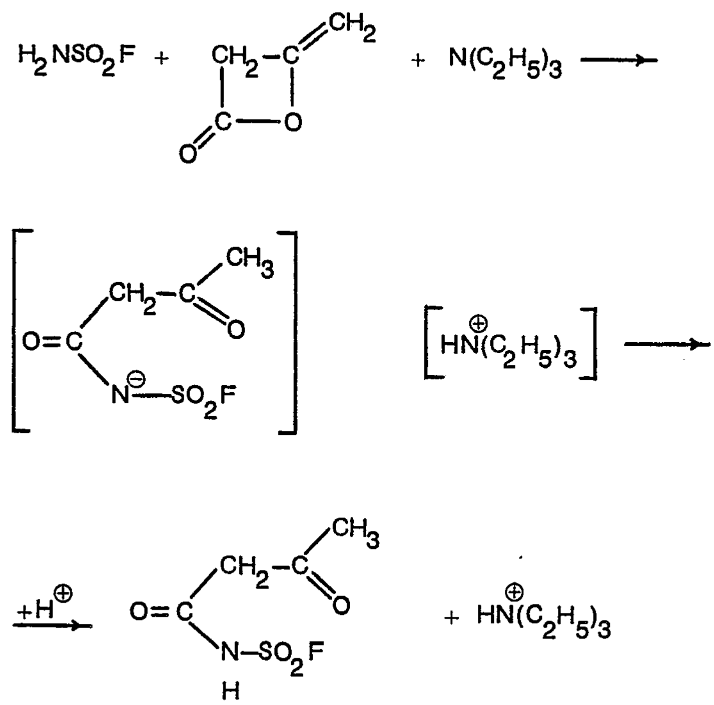

Acetoacetamid-N-sulfofluorid

Das Acetoacetamid-N-sulfofluorid wird dann auf übliche Weise mittels einer Base, z.B. mit methanolischer KOH, zum Sußstoff cyclisiert:

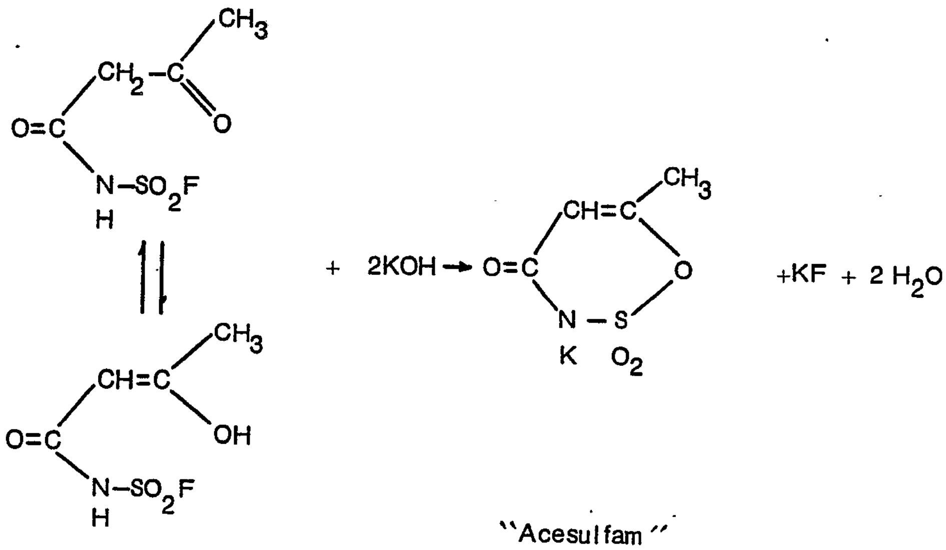


"Acesulfam"

Obwohl die bekannten Verfahren z.T. recht befriedigende Ausbeuten an 6-Methyl-3,4-dihydro-1,2,3-oxathiazin-4-on-2,2-dioxid und dessen nicht-toxischen Salzen liefern, (bis zu ca. 85% d.Th., bezogen auf das Ausgangs-Amidosulfonsäurehalogenid), sind sie wegen der Notwendigkeit des Einsatzes der nicht ganz einfach zugänglichen Ausgangsstoffe Chlor- bzw. Fluorsulfonylisocyanat vor allem für technische Belange noch verbesserungsbedürftig; die Herstellung des Chlor- und Fluor-sulfonylisocyanats erfordert nämlich wegen der z.T. ziemlich unangenehm handzuhabenden Ausgangsmaterialien (HCN, $Cl_2$, $SO_3$ und HF) erhebliche Vorsichtsmaßnahmen und Sicherheitsvorkehrungen. Der Herstellung des Chlor- und Fluor-sulfonylisocyanats liegen folgende Reaktionsgleichungen zugrunde:

$$HCN + Cl_2 \longrightarrow ClCN + HCl$$
$$ClCN + SO_3 \longrightarrow ClSO_2NCO$$
$$ClSO_2NCO + HF \longrightarrow FSO_2NCO + HCl$$

Der Ersatz des Amidosulfofluorids in dem Verfahren gemäß der vorerwähnten DE-OS 24 53 063 etwa durch die wesentlich leichter (z.B. aus $NH_3 + SO_3$) erhältliche Amidosulfonsäure $H_2NSO_3H$ bzw. deren Salze erschien kaum erfolgversprechend, weil nämlich die Umsetzung des Na-Amidosulfonats $H_2NSO_3Na$ mit Diketen in wässrig-alkalischer Lösung überhaupt kein rein isolierbares Umsetzungsprodukt ergibt. Das bei dieser Unsetzung wohl zumindest mit entstandene 1:1-Addukt konnte vielmehr nur in Form des Kupplungsproduktes mit 4-Nitrophenyldiazoniumchlorid als blaßgelber Farbstoff gewonnen werden; vgl. Ber. *83* (1950), S. 551—558, insbesondere S. 555, letzter Absatz vor der Beschreibung der Versuche und S. 558, letzter Absatz:

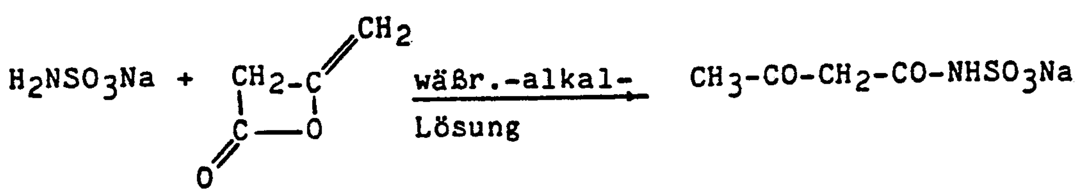


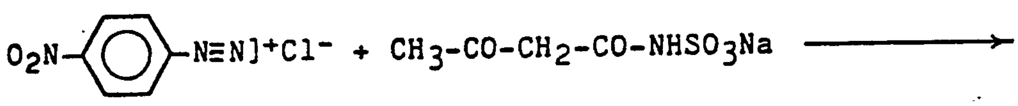


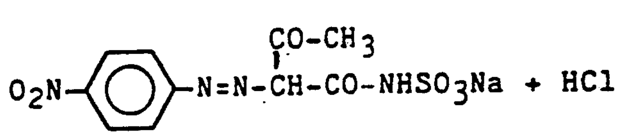

Die Acetoacetamid-N-sulfonsäure ist im übrigen ansonsten nur bzw. auch als Zwischenprodukt bei der Zersetzung des 6-Methyl-3,4-dihydro-1,2,3-oxathiazin-4-on-2,2-dioxids während des Kochens in wässriger Lösung postuliert worden; vgl. die anfangs zitierte Literatur Angew. Chemie (1973) a.a.O.:

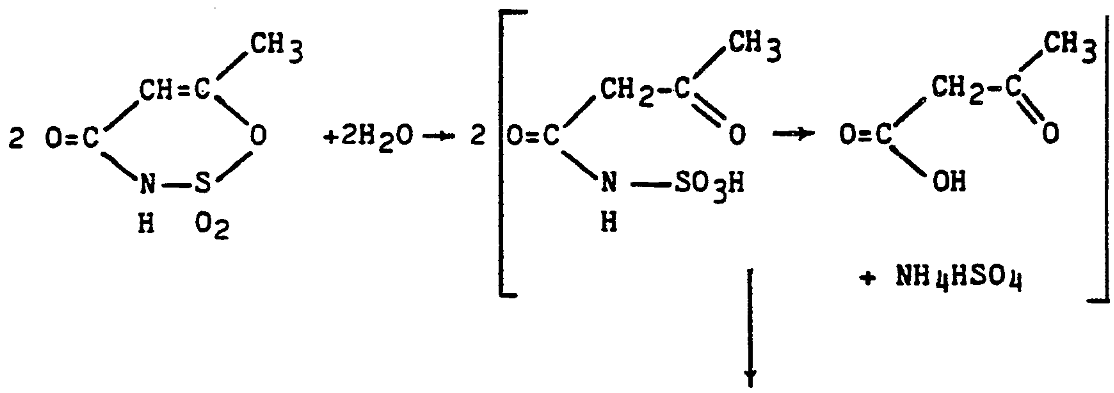

$$2\ CH_3\text{-}CO\text{-}CH_3 + 2\ CO_2 + H_2SO_4 + (NH_4)_2SO_4$$

Wegen der insbesondere infolge der Notwendigkeit des Einsatzes nicht ganz einfach zugänglicher Ausgangsstoffe vor allem für die Durchführung in technischem Maßstab nicht ganz befriedigenden Verfahren des Standes der Technik zur Herstellung von 6-Methyl-3,4-dihydro-1,2,3-oxathiazin-4-on-2,2-dioxid und dessen nicht-toxischer Salze bestand somit die Aufgabe, die bekannten Verfahren entsprechend zu verbessern oder ein neues verbessertes Verfahren zu entwickeln. Diese Aufgabe konnte erfindungsgemäß durch eine Modifikation des Verfahrens gemäß DE—OS 2 453 063 (hauptsächlich Ersatz des Amidosulfofluorids in dem bekannten Verfahren durch Salze der Amidosulfonsäure) mit nachfolgendem Ringschluß des erhaltenen Acetoacetylierungsproduktes mittels $SO_3$ gelöst werden.

Erfindungsgegenstand ist daher ein Verfahren zur Herstellung von 6-Methyl-3,4-dihydro-1,2,3-oxathiazin-4-on-2,2-dioxid und dessen nicht-toxischen Salzen durch

a) Umsetzung eines Amidosulfonsäurederivates mit einer mindestens äquimolaren Menge eines Acetoacetylierungsmittels in einem inerten organischen Lösungsmittel, ggf. in Gegenwart eines Amin- oder Phosphin-Katalysators, zu einem Acetoacetamidderivat und

b) Ringschluß des Acetoacetamidderivats;

das Verfahren ist dadurch gekennzeichnet, daß man in Stufe a) als Amidosulfonsäurederivat ein in dem eingesetzten inerten organischen Lösungsmittel zumindest teilweise lösliches Salz der Amidosulfonsäure und als Acetoacetylierungsmittel Diketen oder Acetoacetylchlorid verwendet, daß man die Umsetzung bei Temperaturen zwischen −30 und +50°C durchführt, daß man das in dieser Stufe gebildete Acetoacetamid-N-sulfonat oder auch die freie Acetoacetamid-N-sulfonsäure in Stufe b) durch die Einwirkung der mindestens äquimolaren Menge von $SO_3$, gegebenenfalls in einem inerten anorganischen oder organischen Lösungsmittel, bei Temperaturen zwischen −70 und +175°C zum Ring des 6-Methyl-3,4-dihydro-1,2,3-oxathiazin-4-on-2,2-dioxids schließt

und daß man das hier in der Säureform anfallende Produkt dann

gegebenenfalls noch in einer Stufe c) mit einer Base neutralisiert.

Die dem Verfahren zugrundeliegenden Reaktionsgleichungen sind (mit Diketen als Acetoacetylierungsmittel):

a)

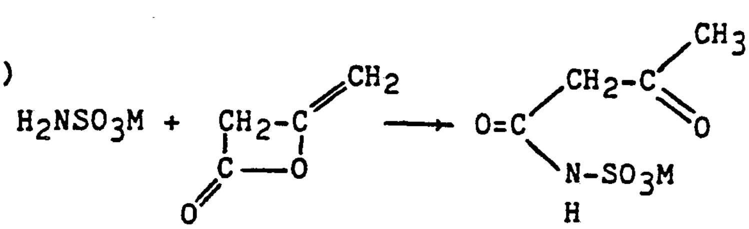

(M =Basenkation)

b)

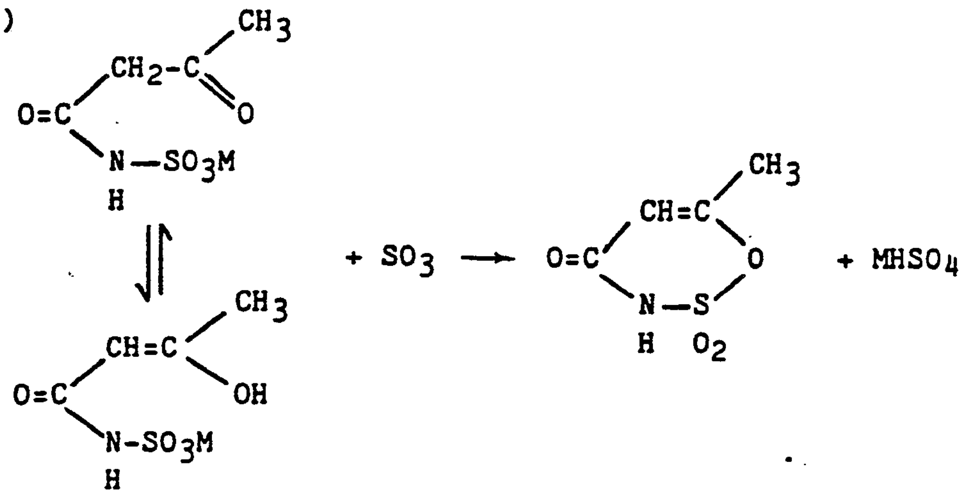

c)

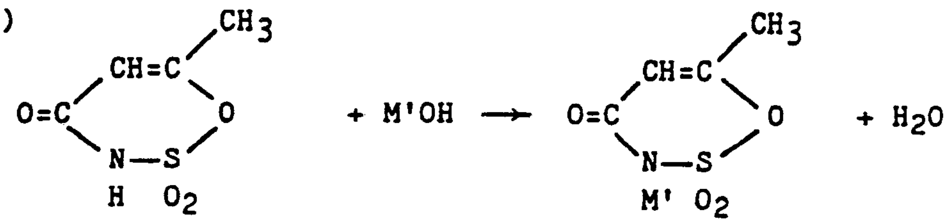

(M' = Basenkation)

Das Verfahren geht von einfach zugänglichen und wohlfeilen Ausgangsstoffen aus und ist außerordentlich einfach durchführbar. Die Ausbeuten liegen

in Stufe a) bei etwa 90 bis 100% d.Th. (bezogen auf das Ausgangs-Amidosulfonat),
in Stufe b) bei etwa 70 bis 95% d.Th. (bezogen auf das Acetoacetamid-N-sulfonat) und
in Stufe c) bei etwa 100% d.Th. (bezogen auf das Oxathiazinon in der Säureform),

so daß für das Gesamtverfahren Ausbeuten zwischen etwa 65 und 95% d.Th. resultieren. Gegenüber den Verfahren des Standes der Technik stellt die Erfindung daher einen erheblichen Fortschritt dar.

Das glatte Gelingen der Reaktion zwischen Amidosulfonat und Acetoacetylierungsmittel zu Acetoacetamid-N-sulfonat gemäß Stufe a) ist außerordentlich überraschend, weil aufgrund der Literaturstelle Ber. *83* (1950) a.a.O., wonach Na-Amidosulfonat mit Diketen in wässrig-alkalischer Lösung offenbar nur ziemlich undefiniert reagiert, zwischen Amidosulfonsäure bzw. deren Salzen und Acetoacetylierungsmitteln kaum mehr eine mit guter Ausbeute an einem ohne Schwierigkeiten rein isolierbaren 1:1-Reaktionsprodukt zu erwarten war.

Ebenso überraschend ist das ausgezeichnete Gelingen des Ringschlusses von Acetoacetamid-N-sulfonat oder auch der freien Sulfonsäure mit $SO_3$ gemäß Stufe b) des Verfahrens, weil die unter Ringschluß erfolgende Wasser- bzw. Basen (MOH)-Abspaltung in dieser Stufe nämlich mit anderen Wasser- bzw. Basen-abspaltenden Mitteln wie z.B. $P_2O_5$, Acetanhydrid, Trifluoressigsäureanhydrid, Thionylchlorid etc. nicht oder jedenfalls praktisch nicht gelingt.

Im einzelnen wird das erfindungsgemäße Verfahren wie folgt ausgeführt.

Stufe a):

Als Acetoacetylierungsmittel können die für Acetoacetylierungen bekannten Verbindungen wie z.B. Acetoacetylchlorid und Diketen eingesetzt werden; bevorzugtes Acetoacetylierungsmittel ist Diketen.

Die Menge des eingesetzten Acetoacetylierungsmittels soll (im Verhältnis zu dem Reaktionspartner Amidosulfonat) mindestens etwa äquimolar sein. Bevorzugt ist der Einsatz eines bis zu etwa 30 Mol-%igen Überschusses, insbesondere eines Überschusses nur bis zu etwa 10 Mol-%. Höhere als etwa 30 Mol-%ige Überschüsse sind möglich, bringen aber keinen Vorteil.

Als inerte organische Lösungsmittel kommen praktisch alle organischen Lösungsmittel in Betracht, welche mit den Ausgangs- und Endstoffen sowie gegebenenfalls den Katalysatoren der Reaktion nicht in unerwünschter Weise reagieren und welche auch die Fähigkeit besitzen, Salze der Amidosulfonsäure zumindest teilweise zu lösen. Folgende organische Lösungsmittel sind daher als hier vorzugsweise in Frage kommend zu nennen:

Halogenierte aliphatische Kohlenwasserstoffe, vorzugsweise solche mit bis zu 4 C-Atomen wie z.B.

Methylenchlorid, Chloroform, 1,2-Dichlorethan, Trichlorethylen, Tetrachlorethylen, Trichlor-fluor-ethylen, etc.;

aliphatische Ketone, vorzugsweise solche mit 3 bis 6 C-Atomen wie z.B. Aceton, Methylethylketon etc.;

Aliphatische Ether, vorzugsweise cyclische aliphatische Ether mit 4 bis 5 C-Atomen wie z.B. Tetrahydrofuran, Dioxan etc.;

niedere aliphatische Carbonsäuren, vorzugsweise solche mit 2 bis 6 C-Atomen wie z.B. Essigsäure, Propionsäure etc.;

aliphatische Nitrile, vorzugsweise Acetonitril;

N-alkylsubstituierte Amide der Kohlensäure und niederen aliphatischer Carbonsäure, vorzugsweise Amide mit bis zu 5 C-Atomen wie z.B. Tetramethylharnstoff, Dimethylformamid, Dimethylacetamid, N-Methylpyrrolidon, etc.;

aliphatische Sulfoxide, vorzugsweise Dimethylsulfoxid, und

aliphatische Sulfone, vorzugsweise Sulfolan

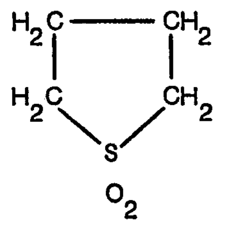

Besonders bevorzugte Lösungsmittel aus der vorstehenden Aufzählung sind Methylenchlorid, 1,2-Dichlorethan, Aceton, Eisessig und Dimethylformamid, vor allem Methylenchlorid.

Die Lösungsmittel können sowohl einzeln als auch in Mischung eingesetzt werden.

Das Mengenverhältnis von Reaktions-Ausgangsstoffen zu Lösungsmittel kann in weiten Grenzen variieren; im allgemeinen liegt das Gewichtsverhältnis bei etwa 1:(2—10). Auch andere Verhältnisse sind jedoch möglich.

Als Amin- und Phosphin-Katalysatoren können im Prinzip alle Amine und Phosphine eingesetzt werden, deren Verwendung als Katalysatoren bei Additionsreaktionen des Diketens bekannt ist. Dies sind hauptsächlich tertiäre Amine und Phosphine mit (noch) nukleophilem Charakter.

Bevorzugt sind im vorliegenden Fall solche tertiären Amine und Phosphine, bei denen auf ein N- bzw. P-Atom bis zu 20, insbesondere nur bis zu 10 C-Atome kommen. Folgende tertiäre Amine sind in beispielhafter Weise zu nennen: Trimethylamin, Triethylamin, Tri-n-propylamin, Triisopropylamin, Tri-n-butylamin, Triisobutylamin, Tricyclohexylamin, Ethyldiisopropylamin, Ethyldicyclohexylamin, N,N-Dimethylanilin, N,N-Diethylanilin, Benzyldimethylamin, Pyridin, substituierte Pyridine wie Picoline, Lutidine, Collidine oder Methylethylpyridine, N-Methylpiperidin, N-Ethylpiperidin, N-Methylmorpholin, N,N-Dimethylpiperazin, 1,5-Diazabicyclo[4.3.0]-nonen-(5), 1,8-Diazabicyclo-[5.4.0]-undecen-(7), ferner Tetramethylhexamethylendiamin, Tetramethylethylendiamin, Tetramethylpropylendiamin, Tetramethylbutylendiamin, oder auch 1,2-Dimorpholylethan, Pentamethyldiethylentriamin, Pentaethyldiethylentriamin, Pentamethyldipropylentriamin, Tetramethyldiaminomethan, Tetrapropyldiaminomethan, Hexamethyltriethylentetramin, Hexamethyltripropylentetramin, Diisobutylentriamin oder Triisopropylentetramin.

Besonders bevorzugtes Amin ist Triethylamin.

Beispielhafte tertiäre Phosphine sind Methyl-diphenylphosphin, Triphenylphosphin, Tributylphosphin, etc.

Die Katalysatormenge beträgt normalerweise bis zu etwa 0,1 Mol pro Mol Amidosulfonat. Höhere Mengen sind möglich, bringen aber kaum mehr Vorteile. Die Reaktions-Stufe a) des erfindungsgemäßen Verfahrens verläuft im Prinzip auch ohne Katalysator; der Katalysator wirkt jedoch reaktionsbeschleunigend und ist daher vorteilhaft.

Die für das Verfahren einzusetzenden Amidosulfonsäuresalze müssen in dem inerten organischen Lösungsmittel zumindest teilweise löslich sein. Diese Forderung wird bevorzugt vom Lithium-, $NH_4$- sowie den primären, sekundären, tertiären und quartären Ammoniumsalzen der Amidosulfonsäure erfüllt. Von den Ammoniumsalzen sind wiederum solche bevorzugt, deren Ammoniumion nicht mehr als etwa 20, insbesondere nicht mehr als etwa 10 C-Atome enthält. Beispielhafte Ammoniumsalze der Amidosulfonsäure sind die Salze mit folgenden Ammonium-ionen:

$$\overset{\oplus}{N}H_4,\ H_3\overset{\oplus}{N}(C_2H_5),\ H_2\overset{\oplus}{N}(n\text{-}C_3H_7)_2,\ H_2\overset{\oplus}{N}(i\text{-}C_3H_7)_2,$$

$$H\overset{\oplus}{N}(CH_3)_3,\ H\overset{\oplus}{N}(C_2H_5)_3,\ H\overset{\oplus}{N}(n\text{-}C_3H_7)_3,\ H\overset{\oplus}{N}(n\text{-}C_4H_9)_3,$$

$$H\overset{\oplus}{N}(CH_3)_2CH_2C_6H_5,\ H\overset{\oplus}{N}(CH_3)_2(C_6H_5),\ \overset{\oplus}{N}(CH_3)_4,\ \overset{\oplus}{N}(C_2H_5)_4,$$

$$\overset{\oplus}{N}(CH_3)_3C_6H_5 \text{ etc.}$$

Besonders bevorzugtes Amidosulfonat ist das Triethylammoniumsalz.

Die Salze werden üblicherweise durch Neutralisation der Amidosulfonsäure mit LiOH, $NH_3$ oder den entsprechenden Aminen bzw. quartären Ammoniumhydroxidlösungen und anschließende Entfernung von Wasser in an sich bekannter Weise erhalten. Vorzugsweise wird die Base in einem stöchiometrischen Überschuß (bezogen auf die Amidosulfonsäure) von bis zu etwa 30 Mol-%, insbesondere nur bis zu etwa 15 Mol-%, zugesetzt. Weiterhin ist es auch bevorzugt, wenn der organische Teil des Ammoniumions mit dem organischen Teil des Aminkatalysators identisch ist (z.B. Verwendung von Triethylammonium-amidosulfonat als Amidosulfonsäuresalz und von Triethylamin als Katalysator). Im Falle der Salze mit $NH_3$ sowie primären bzw. sekundären Aminen, wird die Aminkomponente bevorzugt stöchiometrisch verwendet und als Katalysator ein schwächer basisches tert.-Amin wie z.B. Pyridin zugesetzt.

Die Reaktionstemperatur wird im allgemeinen in einem Bereich zwischen etwa −30 und +50°C, vorzugsweise zwischen etwa 0 und 25°C, gewählt.

Die Reaktion wird normalerweise bei Atmosphärendruck durchgeführt.

Die Reaktionszeit kann innerhalb weiter Grenzen schwanken; sie liegt im allgemeinen zwischen etwa 0,5 und 12 Stunden. Die Umsetzung kann entweder unter Vorlage des Amidosulfonsäuresalzes und Zudosieren von Diketen oder unter Vorlage von Diketen und Zudosieren des Amidosulfonsäuresalzes oder unter Vorlage von Diketen und Amidosulfonsäure und Zudosieren der Base oder etwa auch unter gleichzeitigem Zudosieren beider Reaktanten in den Reaktionsraum erfolgen, wobei das inerte organische Lösungsmittel entweder mit vorgelegt oder zusammen mit den Reaktanten zodosiert werden kann.

Nach Beendigung der Umsetzung wird zur Isolierung des Reaktionsproduktes das Lösungsmittel abdestilliert und der Rückstand (hauptsächlich Acetoacetamid-N-sulfonat) aus einem geeigneten Lösungsmittel wie z.B. Aceton, Methylacetat oder Ethanol, umkristallisiert. Die Ausbeuten liegen bei etwa 90 bis 100% d.Th.

Die Li- und Ammonium-Acetoacetamid-N-sulfonate sind neue Verbindungen. Sie besitzen die Formel

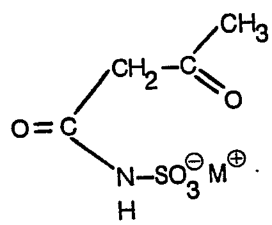

worin $M^{\oplus}$ = $Li^{\oplus}$ oder

$\overset{\oplus}{N}R^1R^2R^3R^4$

mit $R^1$, $R^2$, $R^3$, $R^4$ = unabhängig voneinander = H oder organische Reste,

vorzugsweise = H, $C_1$—$C_8$-Alkyl, $C_6$—$C_{10}$-Cycloalkyl, -Aryl und/oder -Aralkyl.

In den Ammoniumsalzen beträgt die Gesamtzahl der C-Atome im Ammoniumion bevorzugt nicht mehr als etwa 20, insbesondere nicht mehr als eetwa 10.

Aus dem Acetoacetamid-N-sulfonat kann die freie Acetoacetamid-N-sulfonsäure gewünschtenfalls nach üblichen Verfahren gewonnen werden.

Stufe b):

Das in Stufe a) erhaltene Acetoacetamid-N-sulfonat (oder gegebenenfalls auch die freie Säure) wird dann in Stufe b) mit der mindestens etwa äquimolaren Menge $SO_3$, gegebenenfalls in einem inerten anorganischen oder organischen Lösungsmittel, cyclisiert. Das $SO_3$ wird im allgemeinen in einem bis zu etwa 20-fachen, vorzugsweise etwa 3- bis 10-fachen, insbesondere etwa 4- bis 7-fachen molaren Überschuß, bezogen auf das Acetoacetamid-N-sulfonat (oder die freie Säure) eingesetzt. Es kann dem Reaktionsansatz sowohl in fester oder flüssiger Form als auch durch Einkondensation von $SO_3$-Dampf zugegeben werden. Üblicherweise wird jedoch eine $SO_3$-Lösung in konzentrierter Schwefelsäure, flüssigem $SO_2$ oder einem inerten organischen Lösungsmittel verwendet. Auch der Einsatz von $SO_3$-abspaltenden Verbindungen ist möglich.

Die Reaktion kann zwar im Prinzip ohne Lösungsmittel durchgeführt werden, doch ist die Durchführung in einem inerten anorganischen oder organischen Lösungsmittel bevorzugt. Als solche inerten anorganischen oder organischen Lösungsmittel kommen Flüssigkeiten in Frage, die mit $SO_3$ sowie den Reaktions-Ausgangs- und -Endstoffen nicht in unerwünschter Weise reagieren. Wegen der erheblichen Reaktionsfähigkeit insbesondere des $SO_3$ kommen daher hier nur relativ wenige Lösungsmittel in Frage. Bevorzugte Lösungsmittel sind:

Anorganische Lösungsmittel: flüssiges $SO_2$;

organische Lösungsmittel: halogenierte aliphatische Kohlenwasserstoffe, vorzugsweise mit bis zu 4 C-Atomen wie z.B. Methylenchlorid, Chloroform, 1,2-Dichlorethan, Trichlorethylen, Tetrachlorethylen, Trichlor-fluorethylen etc.;

Kohlensäureester mit niederen aliphatischen Alkoholen, vorzugsweise mit Methanol oder Ethanol;

Nitroalkane, vorzugsweise mit bis zu 4 C-Atomen, insbesondere Nitromethan;
alkylsubstituierte Pyridine, vorzugsweise Collidin; und
aliphatische Sulfone, vorzugsweise Sulfolan.

Die organischen Lösungsmittel können sowohl einzeln als auch in Mischung eingesetzt werden.

Besonders bevorzugte Lösungsmittel sind flüssiges $SO_2$ und Methylenchlorid.

Die Menge des eingesetzten inerten Lösungsmittels ist nicht kritisch. Wenn ein Lösungsmittel eingesetzt wird, soll lediglich eine ausreichende Lösung der Reaktanten gewährleistet sein; nach oben ist die Menge des Lösungsmittels von Wirtschaftlichkeitserwägungen begrenzt.

In einer bevorzugten Ausführungsform des erfindungsgemäßen Verfahrens wird sowohl in Stufe a) als auch in Stufe b) das gleiche Lösungsmittel verwendet; dies ist vorzugsweise ein halogenierter aliphatischer Kohlenwasserstoff, insbesondere Methylenchlorid. In diesem Fall kann nämlich die in Stufe a) erhaltene Lösung ohne Isolierung des Acetoacetamid-N-sulfonats gleich für Stufe b) eingesetzt werden.

Die Reaktionstemperatur in Stufe b) liegt normalerweise zwischen etwa −70 und +175°C, vorzugsweise zwischen etwa −40 und +10°C.

Wie Stufe a), wird auch Stufe b) normalerweise nur bei Atmosphärendruck durchgeführt.

Die Reaktionszeit kann bis zu etwa 10 Stunden betragen.

Man kann die Reaktionsführung in der Weise gestalten, daß man das Acetoacetamid-N-sulfonat (oder auch die freie Säure), gegebenenfalls in Lösung vorlegt, und $SO_3$, gegebenenfalls in gelöster Form, zudosiert oder beide Reaktionspartner gleichzeitig in den Reaktionsraum schleust oder $SO_3$ vorlegt und das Acetoacetamid-N-sulfonat (oder die freie Säure) zuführt.

Bevorzugt wird ein Teil des $SO_3$, gegebenenfalls in Lösung, vorgelegt und dan entweder kontinuierlich oder portionsweise Acetoacetamid-N-sulfonat (oder die freie Säure) als auch $SO_3$, gegebenenfalls in gelöster Form, zudosiert.

Die Aufarbeitung geschieht auf übliche Weise. Im bevorzugten Fall der Verwendung von Methylenchlorid als Reaktionsmedium kann die Aufarbeitung beispielsweise wie folgt vorgenommen werden: Man versetzt die $SO_3$-haltige Lösung mit der (bezogen auf $SO_3$) etwa 10-fachen molaren Menge Eis oder Wasser. Hierdurch wird eine Phasentrennung hervorgerufen: Das gebildete 6-Methyl-3,4-dihydro-1,2,3-oxathiazin-4-on-2,2-dioxid befindet sich hauptsächlich in der organischen Phase. Die noch in der wässrigen Schwefelsäure befindlichen Anteile können durch Extraktion mit einem organischen Lösungsmittel wie z.B. mit Methylenchlorid oder einem organischen Ester gewonnen werden.

Oder man destilliert nach der Zugabe von Wasser das Reaktionslösemittel ab und extrahiert das in der Reaktionsschwefelsäure zurückbleibende 6-Methyl-3,4-dihydro-1,2,3-oxathiazin-4-on-2,2-dioxid mit einem besser geeigneten Lösemittel. Als Lösemittel eignen sich solche, die gegen Schwefelsäure hinreichend stabil sind und ein zufriedenstellendes Lösevermögen aufweisen; außerdem sollte das Reaktionsprodukt in dem Lösemittelsystem einen für die Isolierung güngstigen Verteilungskoeffizienten besitzen. Neben halogenierten Kohlenwasserstoffen eignen sich Kohlensäureester wie z.B. Dimethylcarbonat, Diethylcarbonat und Ethylencarbonat oder Ester organischer Monocarbonsäuren wie z.B. Isopropylformiat und Isobutylformiat, Ethylacetat, Isopropylacetat, Butylacetat, Isobutylacetat und Neopentylacetat oder Dicarbonsäureester oder mit Wasser nicht mischbare Amide wie z.B. Tetrabutylharnstoff. Besonders bevorzugt werden Isopropalacetat und Isobutylacetat.

Die vereinigten organischen Phasen werden z.B. mit $Na_2SO_4$ getrocknet und eingeengt. Bei der Extraktion ggf. mitgerissene Schwefelsäure kann man auch durch gezielte Zugabe wäßriger Lauge zur organischen Phase entfernen Hierzu gibt man soviel verdünnte wäßrige Lauge zur organischen Phase, bis in der wäßrigen Phase der pH-Wert erreicht ist, den das reine 6-Methyl-3,4-dihydro-1,2,3-oxathiazin-4-on-2,2-dioxid bei gleicher Konzentration im gleichen Zweiphasensystem aus Extraktionsmittel und Wasser anzeigt. Falls die Gewinnung der freien Verbindung beabsichtigt ist, wird diese noch auf übliche Weise gereinigt (vorzugsweise durch Umkristallisation). Die Ausbeute liegt zwischen etwa 70 und 95% d.Th., bezogen auf das Acetoacetamid-Nsulfonat (bzw. die freie Säure).

Wenn jedoch die Gewinnung eines nicht-toxischen Salzes des 6-Methyl-3,4-dihydro-1,2,3-oxathiazin-4-on-2,2-dioxids beabsichtigt ist, folgt noch die Neutralisationsstufe c).

Dazu wird die in Stufe b) in der Säureform anfallende Oxathiazinon-Verbindung auf übliche Weise mit einer entsprechenden Base neutralisiert. Zu diesem Zweck werden beispielsweise die am Ende der Stufe b) vereinigten, getrockneten und eingeengten organischen Phasen in geeigneten organischen Lösungsmitteln, wie z.B. Alkoholen, Ketonen, Estern oder Ethern oder auch in Wasser mit einer entsprechenden Base — vorzugsweise mit einer Kaliumbase wie z.B. KOH, $KHCO_3$, $K_2CO_3$, K-Alkoholate etc., neutralisiert. Oder die Oxathiazinon-Verbindung wird aus der gereinigten organischen Extraktionsphase (Stufe b) mit wäßriger Kaliumbase direkt extraktiv neutralisiert. Das Oxathiazinon-Salz fällt dann, gegebenenfalls nach Einengen der Lösung, in kristalliner Form aus und kann zur Reinigung noch umkristallisiert werden.

Die Neutralisationsstufe verläuft mit praktisch 100%iger Ausbeute.

Sowohl das aus den Verfahrensstufen a), b) und c) bestehende erfindungsgemaße Gesamtverfahren als auch die einzelnen Verfahrensstufen a) und b) sind neu und von erheblichem Vorteil.

Die folgenden Beispiele sollen der weiteren Erläuterung der Erfindung dienen. Nach den (Erfindungs-)-Beispielen für die Durchführung der Verfahrensstufen a), b) und c) folgt noch ein Vergleichsbeispiel, welches zeigt, daß Acetoacetamid-N-sulfonate mit anderen Wasser- bzw. Basenabspaltenden Mitteln als $SO_3$ — hier $P_2O_5$ — nicht cyclisieren:

A) *Beispiele für die Durchführung der Verfahrensstufe a:*

Beispiel 1

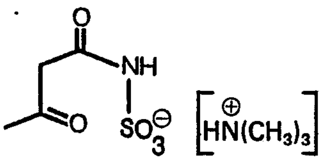

Trimethylammonium-(acetoacetamid-N-sulfonat)

Es wurden 9,7 g (0,1 Mol) Amidosulfonsäure zu einer Lösung von 12 ml (0,125 Mol) Trimethylamin in 100 ml Eisessig gegeben und gerührt bis alles gelöst war. Dann wurden 8 ml (0,104 Mol) Diketen bei 25—30°C unter Kühlung zugetropft. Nach 16 Stdn. wurde das Reaktionsprodukt durch langsame Zugabe von Ether ausgefällt und abgesaugt.

22 g (92%), Fp. 101°C.

NMR (DMSO $d_6$) δ 2,2 ($CH_3-\underset{\underset{O}{\|}}{C}$), 2,8 ($\overset{\oplus}{N}-CH_3$), 3,45 ($-CH_2$)

JR (KBr) 1045, 1240, 1470, 1660, 1720 $cm^{-1}$

Beispiel 2

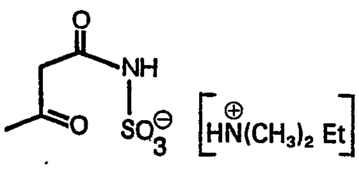

Dimethyl-ethyl-ammonium-(acetoacetamid-N-sulfonat)

Zu 80 g (0,825 Mol) Amidosulfonsäure in 500 ml Eisessig suspendiert wurden unter Kühlung 80 g (1,096 Mol) Dimethylethylamin getropft. Als alles gelöst war, wurden unter Kühlung bei 25—35°C 80 ml (1,038 Mol) Diketen zugegeben. Nach 16 Stdn. wurde eingedampft und der Rückstand mit Aceton verrührt, wobei Kristallisation erfolgte.

110 g (43%), Fp. 73—75°C

Aus der Mutterlauge wurde der Rest des Reaktionsproduktes 128 g (50%) als Sirup gewonnen.

NMR ($CDCl_3$) δ 1,35 ($CH_3$), 2,2 ($CH_3-\underset{\underset{O}{\|}}{C}$), 2,8 ($\overset{\oplus}{N}-CH_3$), 3,5 ($-CH_2-\underset{\underset{O}{\|}}{C}$)

JR (KBr) 1050, 1240, 1475, 1690, 1730 $cm^{-1}$

Beispiel 3

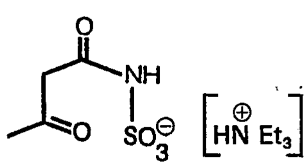

Triethylammonium-(acetoactamid-N-sulfonat)

9,7 g (0,1 Mol) Amidosulfonsäure wurden in 100 ml Methylenchlorid mit 16 ml (0,12 Mol) Triethylamin in Lösung gebracht. Danach wurden bei 0°C 8 ml (0,104 Mol) Diketen zugetropft. 2 Stdn. wurde bei 0°C und 2 Stdn. bei Raumtemperatur nachgerührt. Dann wurde durch Hexanzugabe das Reaktionsprodukt ausgefällt und der verbleibende Sirup mit weiterem Hexan gewaschen. Nach dem Trocknen im Vakuum verblieben 27—28 g (95,7—99%); nach längerem Stehen begann der Sirup zu kristallisieren.

NMR ($CDCl_3$) δ 1,33 ($-CH_3$), 2,2 ($CH_3-\underset{\underset{O}{\|}}{C}$), 3,2 ($N-CH_2$), 3,5 ($-CH_2-\overset{\overset{O}{\|}}{C}$)

JR (neat) 1040, 1230, 1450, 1650, 1670 $cm^{-1}$

In analoger Weise wie Beispiel 3 wurden die folgenden Beispiele 4—7 durchgeführt; das Ergebnis war:

Beispiel 4

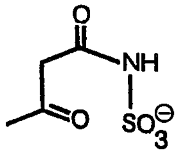 $[HN^{\oplus}(n\text{-}Prop.)_3]$ Tri-(n-propyl)-ammonium-acetoacetamid-N-sulfonat

Ausbeute: 92—97%
NMR ($CDCl_3$) δ 2,3 ($—CH_3—\underset{O}{\underset{\|}{C}}$), 3,6 ($—CH_2—\underset{O}{\underset{\|}{C}}$)
JR ($CH_2Cl_2$) 1040, 1260, 1420, 1700, 1740 $cm^{-1}$

Beispiel 5

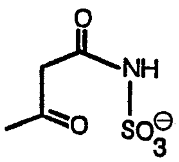 $[HN^{\oplus}(n\text{-}Bu)_3]$ Tri-(n-Butyl)-ammonium)acetoacetamid-N-sulfonat)

Ausbeute: 91—96%
NMR ($CDCl_3$) δ 2,25 ($CH_3—\overset{O}{\overset{/\!/}{C}}$), 3,5 ($—CH_2—\overset{O}{\overset{/\!/}{C}}$)
JR ($CH_2Cl_2$) 1040, 1250, 1420, 1700, 1740 $cm^{-1}$

Beispiel 6

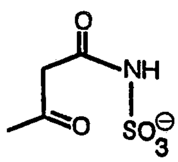 $[HN^{\oplus}(CH_3)_2Benzyl]$ Dimethyl-Benzyl-ammonium(acetoacetamid-N-sulfonat)

Ausbeute: 92—97%
NMR ($CDCl_3$) δ 2,2 ($COCH_3$), 2,75 ($\overset{\oplus}{N}—CH_3$), 3,5 ($—CH_2—\underset{O}{C}$), 4,3 ($\overset{\oplus}{N}—CH_2—Ar$), 7,35 (Ar),
JR ($CH_2Cl_2$) 1040, 1260, 1270, 1430, 1470, 1700, 1740 $cm^{-1}$

Beispiel 7

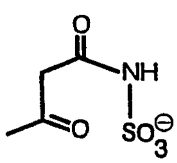 $[HN^{\oplus}(i\text{-}Propy)_2Et]$ Diisopropyl-ethyl-ammonium(acetoacetamid-N-sulfonat)

Ausbeute: 91—95%
NMR ($CDCl_3$) δ 1,3 u. 1,4 ($—CH_3$), 2,2 ($COCH_3$), 3,5 ($CH_2—CO$)
JR ($CH_2Cl_2$) 1040, 1210, 1250, 1420, 1700, 1740 $cm^{-1}$

Beispiel 8

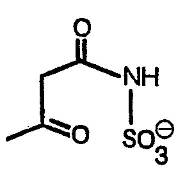 $[HN^{\oplus}\,Et_3]$ Triethylammonium-(acetoacetamid-N-sulfonat)

9,7 g (0,1 Mol) Amidosulfonsäure wurden in 100 ml Aceton suspendiert und 16 ml (0,12 Mol) Triethylamin zugegeben. Als fast alles gelöst war, wurden bei 0°C 8 ml (0,104 Mol) Diketen zugetropft. Danach wurde unter Rühren bei Raumtemperatur ausreagieren gelassen, wobei alles in Lösung ging. Nach 16 Std. wurde das Reaktionsprodukt mit Hexan als Sirup ausgefällt und dieser durch Rühren mit Hexan noch gereinigt. Nach dem Trocknen im Vakuum verblieben 27—28 g (95,7—99%) Sirup, der beim Stehen langsam kristallisierte.

NMR ($CDCl_3$) δ 1,3 ($—CH_3$), 2,2 ($CH_3—C$), 3,55 ($—CH_2—C$),
                                  ||                    ||
                                  O                     O

JR (neat) 1040, 1230, 1450, 1670 $cm^{-1}$

Beispiel 9

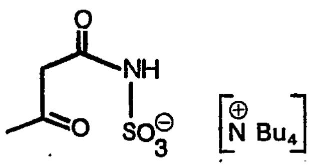
Tetrabutylammonium-(acetoacetamid-N-sulfonat)

15,5 g (0,16 Mol) Amidosulfonsäure wurden in 10 ml Methanol und 50 ml Wasser mit 105 ml (0,16 Mol) einer 40%igen wäßrigen Tetrabutylammoniumhydroxidlösung versetzt. Danach wurde zur Trockne eingedampft. Der Rückstand wurde in 100 ml Methylenchlorid gelöst und mit Triethylamin auf einen pH von 9—10 gebracht. Danach wurden 10 ml Diketen zugetropft. Nach 12 Stdn. wurde der pH erneut auf 9—10 eingestellt und die Diketenzugabe wiederholt. 16 Stdn. später wurde eingedampft, wobei der Rückstand kristallisierte. Der Kristallbrei wurde abgesaugt und mit Ethylacetat und Ether gewaschen.

34,6 g (52%) Fp.: 97—98°C

NMR ($CDCl_3$) δ 1,33 ($—CH_3$), 2,2 ($COCH_3$), 3,2 ($CH_2—C$), 3,5 ($CH_3—C$),
                                                       ||          ||
                                                       O           O

JR ($CH_2Cl_2$) 890, 1040, 1255, 1410 $cm^{-1}$

Beispiel 10

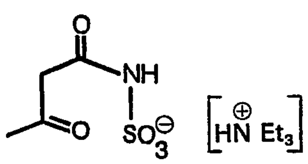
Triethylammonium-(acetoactamid-N-sulfonat)

19,4 g (0,2 Mol) Amidosulfonsäure und 15,4 ml (0,2 Mol) Diketen wurden in 200 ml Methylenchlorid bei 0°C vorgelegt. Innerhalb 45 Min. wurde unter Kühlung und Rühren 29 ml (0,21 Mol) Triethylamin zutropfen gelassen. Anschließend wurde 30 Min. bei 0°C nachgerührt und dann die Reaktionsmischung bei Raumtemperatur über Nacht stehengelassen. Nach dem Abdampfen des Lösungsmittels und dem Trocknen im Vakuum wurde das Reaktionsprodukt als Sirup erhalten. Die Kristallisation erfolgte aus Aceton.

53—56 g (94—99%); Fp. 55—58°C

NMR ($CDCl_3$) δ 1.33 ($—CH_3$), 2,2 ($CH_3—C$), 3,2 ($N—CH_2$), 3,5 ($CH_2—C$)
                                    ||                          ||
                                    O                           O

JR (neat) 1040, 1230, 1450, 1670 $cm^{-1}$

Beispiel 11

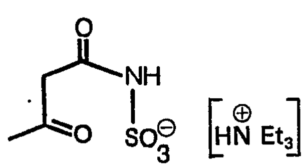
Triethylammonium-(acetoacetamid-N-sulfonat)

19,4 g (0,2 Mol) Amidosulfonsäure, 15,4 ml (0,2 Mol) Diketen und 1,14 ml (0,02 Mol) Eisessig wurden in 100 ml Methylenchlorid bei 0°C vorgelegt. Innerhalb 45 Min. wurde unter Kühlung und Rühren 29 ml (0,21 Mol) Triethylamin zutropfen gelassen. Anschließend wurde 30 Min. bei 0°C nach gerührt und dann die Reaktionsmischung bei Raumtemperatur über Nacht stehengelassen. Nach dem Abdampfen des

Lösungsmittels wurde der Rückstand mit Diethylether gewaschen und dann im Vakuum getrocknet. Die Kristallisation erfolgte aus Aceton.

52—5 g (92—97,5%) Fp. 55—58°C

NMR ($CDCl_3$) δ 1,33 (—$CH_3$), 2,2 ($CH_3$—C), 3,5 ($CH_2$—C)
‖ ‖
O O

JR (neat) 1040, 1230, 1450, 1670 $cm^{-1}$

Beispiel 12

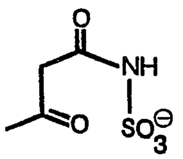
$\left[H\overset{\oplus}{N}(CH_3)_2C_6H_5\right]$ Dimethyl-phenyl-ammonium-(acetoacetamid-N-sulfonat)

Zu 9,7 g (100 mMol) Amidosulfonsäure in 100 ml Eisessig wurden 15,1 ml (120 mMol) N,N-Dimethylanilin gegeben und gerührt bis alles gelöst war. Dann wurden 8 ml (104 mMol) Diketen zugegeben. Nach 16 Stdn. kamen noch einmal 2 ml Diketen zur Lösung. Als das Diketen verschwunden war, wurde eingeengt und das Produkt durch Verrühren mit Äther ausgefällt.

Ausbeute: 88—92%

NMR ($CDCl_3$) δ 2,2 ($COCH_3$), 3,5 ($CH_2$—C)
‖
O

JR ($CH_2Cl_2$) 1040, 1250, 1430, 1700, 1740 $cm^{-1}$

Beispiel 13

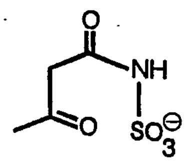
$[NH_4^{\oplus}]$ Ammonium-(acetoacetamid-N-sulfonat)

Zu einer Suspension von 11,4 g (100 mMol) Ammoniumamidosulfonat in 100 ml Eisessig wurde unter gutem Rühren 10 ml Diketen und 1 ml Pyridin gegeben. Nach 17 Stunden saugte man das Endprodukt ab.

17 g = 86% Zersetzung ab etwa 125°C.

Beispiel 14

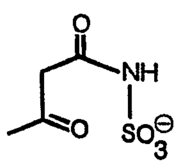
$[H_2\overset{\oplus}{N}(iProp)_2]$ Diisopropylammonium-(acetoacetamid-N-sulfonat)

19,4 g (0,2 Mol) Amidosulfonsäure wurden in 200 ml $CH_2Cl_2$ mit 28 ml (0,2 Mol) Diisopropylamin neutralisiert. Nach der Zugabe von 0,81 ml (10 mMol) Pyridin tropfte man bei 0°C 15,4 ml (0,2 Mol) Diketen zu. Anschließend wurde 30 Minuten bei 0°C nachgerührt und dann die Reaktionsmischung bei Raumtemperatur über Nacht stehengelassen. Nach dem Abdanpfen des Lösungsmittels und dem Trocknen im Vakuum wurde das Reaktionsprodukt als Lösung erhalten.

45—48 g = 80—85%

IR (neat) 1040, 1280, 1450, 1670 $cm^{-1}$

Beispiel 15

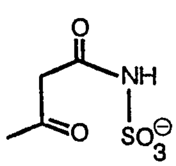
$[H_3\overset{\oplus}{N}$ tert.Bu] tert.-Butylammonium-(acetoacetamid-N-sulfonat)

19,4 g (0,2 Mol) Amidosulfonsäure wurden in 100 ml DMF mit 21 ml (0,2 Mol) tert.-Butylamin

neutralisiert. Nach der Zugabe von 0,81 ml (10 mMol) Pyridin tropfte man bei 15°C 15,4 ml (0,2 Mol) Diketen zu. Anschließend wurde 3. Stunden bei Raumtemperatur nachgerührt. Zur Aufarbeitung wurde das Reaktionsprodukt mit 500 ml Diethylether ausgefällt. Zur Reinigung wurde der Sirup mit Aceton verrührt.

Ausbeute: 42 g = 83%

IR (neat) 1035, 1230, 1450, 1670 $cm^{-1}$

B] *Beispiele für die Durchführung der Verfahrensstufen b) und c):*

Beispiel 1

Zu 8 ml (200 mMol) flüssigem $SO_3$ in 100 ml $CH_2Cl_2$ wurden bei −30°C unter gutem Rühren innerhalb von 60 Minuten 12,7 g (50 mMol) Dimethyl-ethyl-ammonium-acetoacetamid-N-sulfonat in 110 ml Methylenchlorid getropft. 30 Minuten später kamen 50 ml Ethylacetat und 50 g Eis zur Lösung. Die organische Phase wurde abgetrennt, die wässrige noch zweimal mit Ethylacetat extrahiert. Die vereinigten organischen Phasen wurden nach dem Trocknen über Natriumsulfat eingeengt und in Methanol gelöst. Beim Neutralisieren der Lösung mit methanolischer KOH fiel das Kaliumsalz des 6-Methyl-3,4-dihydro-1,2,3-oxathiazin-4-on-2,2-dioxids aus.

7,3 g = 73%.

Beispiel 2

Zu 8 ml (200 mMol) flüssigem $SO_3$ in 50 ml $SO_2$ wurden bei −30°C unter gutem Rühren innerhalb von 60 Minuten 12,7 g (50 mMol) Dimethyl-ethylammonium-acetoacetamid-N-sulfonat in 110 ml $CH_2Cl_2$ getropft. 30 Minuten später kamen nach dem Abdampfen des $SO_2$ 50 ml Ethylacetat und 50 g Eis zur Lösung. Die organische Phase wurde abgetrennt, die wässrige noch zweimal mit Ethylacetat extrahiert. Die vereinigten organischen Phasen wurden nach dem Trocknen über Natriumsulfat eingeengt und in Methanol gelöst. Beim Neutralisieren der Lösung mit methanolischer KOH fiel das Kaliumsalz des 6-Methyl-3,4-dihydro-1,2,3-oxathiazin-4-on-2,2-dioxids aus.

8,3 g = 83%.

Beispiel 3

Zu 12 ml (300 mMol) flüssigem $SO_3$ in 100 ml $CH_2Cl_2$ wurden bei −30°C unter gutem Rühren innerhalb von 60 Minuten 12,7 g (50 mMol) Dimethyl-ethylammonium-acetoacetamid-N-sulfonat in 110 ml $CH_2Cl_2$ getropft. 30 Minuten später kamen 50 ml Ethylacetat und 50 g Eis zur Lösung. Die organische Phase wurde abgetrennt, die wässrige Phase noch zweimal mit Ethylacetat extrahiert. Die vereinigten organischen Phasen wurden nach dem Trocknen über Natriumsulfat eingeengt und in Methanol gelöst. Beim Neutralisieren der Lösung mit methanolischer KOH fiel das Kaliumsalz des 6-Methyl-3,4-dihydro-1,2,3-oxa-thiazin-4-on-2,2-dioxids aus.

7,6 g = 76%

Beispiel 4

Zu 4 ml (100 mMol) flüssigem $SO_3$ in 100 ml $CH_2Cl_2$ wurden bei −30°C unter gutem Rühren innerhalb von 20 Minuten 4,24 g (16,7 mMol) Dimethyl-ethylammonium-acetoacetamid-N-sulfonat in 35 ml $CH_2Cl_2$ getropft. Danach kamen 4 ml (100 mMol) $SO_3$ zur Lösung, worauf erneut innerhalb von 20 Minuten unter gutem Rühren bei −30°C 4,24 g (16,7 mMol) Dimethyl-ethyl-ammonium-acetoacetamid-N-sulfonat in 35 ml $CH_2Cl_2$ zugetropft wurden. Anschließend wurde die Zugabe von 4 ml (100 mMol) $SO_3$ wiederholt. Dann wurden bei −30°C unter gutem Rühren innerhalb von 20 Minuten 4,24 g (16,6 mMol) Dimethylethyl-ammonium-acetoacetamid-N-sulfonat in 35 ml $CH_2Cl_2$ zugetropft. 20 Minuten später wurde wie in Beispiel 1 aufgearbeitet.

8,7 g = 87%.

Beispiel 5

Zu 2,4 ml (60 mMol) $SO_3$ in 100 ml $CH_2Cl_2$ wurden bei −25°C unter gutem Rühren innerhalb von 60 Minuten 12,7 g (50 mMol) Dimethyl-ethyl-ammonium-acetoacetamid-N-sulfonat in 110 ml $CH_2Cl_2$ zugetropft. Gleichzeitig wurde nach 12, 24, 36, 48 Minuten jeweils 2,4 ml (60 mMol) $SO_3$ zugegeben. 20 Minuten später wurde aufgearbeitet wie in Beispiel 1.

8,8 g = 88%.

Beispiel 6

Es wurde wie in Beispiel 5 gearbeitet, nur daß 2,4 ml (60 mMol) $SO_3$ in 50 ml $SO_2$ zu Beginn vorgelegt wurden.

8,8 g = 88%.

Beispiel 7

12,8 g (160 mMol) festes $SO_3$ wurden in 150 ml $CH_2Cl_2$ gelöst. Nach Kühlen der Lösung auf −45/−55°C wurden innerhalb von 60 Minuten 8,4 g (26 mMol) Tripropylammonium-acetoacetamid-N-sulfonat in 25 ml $CH_2Cl_2$ zugetropft. Nach 4 Stunden bei −45/−55°C wurde wie in Beispiel 1 aufgearbeitet.

2,8 g = 54%.

Bei den Beispielen 8—12 wurden die Reaktionslösungen aus der Umsetzung von Diketen, Amidosulfonsäure und Triethylamin direkt eingesetzt.

Beispiel 8

Zu 20 ml (500 mMol) flüssigem $SO_3$ in 500 ml $CH_2Cl_2$ wurden bei −30°C unter gutem Rühren 125 ml Triethylammonium-(acetoacetamid-N-sulfonat)-Lösung (0,1 Mol; $CH_2Cl_2$) innerhalb 60 Minuten zugetropft. Nach weiteren 60 Minuten bei −30°C wurde wie in Beispiel 1 aufgearbeitet.

17,1 g = 85%.

Beispiel 9

125 ml Triethylammonium-(acetoacetamid-N-sulfonat)-Lösung (0,1 Mol; $CH_2Cl_2$) wurden in 250 ml $Ch_2Cl_2$ bei −30°C vorgelegt. Innerhalb von 60 Minuten wurden 20 ml (500 mMol) flüssiges $SO_3$ gelöst in 250 ml $CH_2Cl_2$ zugesetzt. Nach weiteren 60 Minuten bei −30°C wurde wie in Beispiel 1 aufgearbeitet.

14,9 g = 74%.

Beispiel 10

Zu 4,8 ml (120 mMol) flüssigem $SO_3$ in 500 ml $CH_2Cl_2$ wurden bei −25°C innerhalb von 60 Minuten 125 ml Triethylammonium-(acetoacetamid-N-sulfonat)-Lösung (0,1 Mol; $Ch_2Cl_2$) zugetropft. In Abständen von je 12 Minuten wurden vier weitere Portionen von je 4,8 ml (120 mMol) flüssigem $SO_3$ zugesetzt. Nach weiteren 60 Minuten bei −25°C wurde wie in Beispiel 1 aufgearbeitet.

18,3 g = 91%.

Beispiel 11

50 ml $CH_2Cl_2$ wurden bei −30°C vorgelegt. Unter guter Kühlung und Rührung ließ man gleichzeitig und gleichmäßig eine Lösung von 28,1 g (0,1 Mol) Triethylammonium(acetoacetamid-N-sulfonat) in 50 ml $CH_2Cl_2$, und 24 ml flüssiges $SO_3$ in 50 ml $CH_2Cl_2$ innerhalb 30 Min. zutropfen. Nach weiteren 30 Min. bei −25°C bis −30°C wurden bei der gleichen Temperatur 110 ml Wasser vorsichtig zugetropft. Dann destillierte man das $CH_2Cl_2$ ab und extrahierte das Reaktionsprodukt mit 80 ml i-Butylacetat. Die organische Phase wurde dann mit 20 ml Wasser versetzt und unter guter Rührung mit 4 n KOH auf pH 0,84—0,87 (pH-Meter, Glaselektrode: Ingold 405-60-S7) gestellt. Nach dem Abtrennen und Extrahieren der wäßrigen Phase mit 20 ml i-Butylacetat wurden zu den vereinigten i-Butylacetatphasen 15 ml Wasser gegeben und unter Rühren mit 4 n KOH bis pH 5—7 neutralisiert. Das zum Teil ausgefallene K-Salz wurde abgesaugt und anschließend mit der wäßrigen Phase des Filtrats vereinigt. Abdampfen des Wassers im Vakuum lieferte 18,1 g = 90% Sußstoff.

Beispiel 12

50 ml $CH_2Cl_2$ wurden bei −30°C vorgelegt. Anschließend wurden gleichzeitig und gleichmäßig eine Lösung von 28,1 g (0,1 Mol) Triethylammonium-(acetoacetamid-N-sulfonat) in 50 ml $CH_2Cl_2$ und 24 ml flüssiges $SO_3$ in 50 ml $CH_2Cl_2$ unter kräftiger Kühlung (Isopropanol/Trockeneis) zulaufen gelassen. Sofortiges Aufarbeiten wie in Beispiel 11 (Extraktionsmittel: Isopropylacetat) lieferte 17,9 g = 89% Süßstoff.

Beispiel 13

12,4 ml 60%iges Oleum (200 mMol $SO_3$) wurden in 200 ml $CH_2Cl_2$ bei −25°C vorgelegt. Innerhalb von 30 Minuten wurden 62,5 ml Triethylammonium-(acetoacetamid-N-sulfonat)-Lösung (50 mMol); $CH_2Cl_2$) zugetropft. Nach weiteren 60 Minuten bei −25°C wurde wie in Beispiel 1 aufgearbeitet.

4,7 g = 47%.

Beispiel 14

200 ml Collidin wurden bei −30°C vorsichtig mit 8 ml (200 mMol) flüssigem $SO_3$ versetzt. Anschließend wurden 16,2 g (50 mMol) Tripropylammonium-(acetoacetamid-N-sulfonat) hinzugegeben und die Reaktionsmischung 20 Stunden auf ca. 100°C erwärmt. Der größte Teil des Collidins wurde dann im Vakuum abdestilliert und der Rückstand in Ethylacetat aufgenommen. Nach dem Ansäuern mit Schwefelsäure wurde die wässrige Phase mit Ethylacetat gut extrahiert. Die organischen Phasen wurden über $Na_2SO_4$ getrocknet und im Vakuum eingedampft. Der Rückstand wurde in Methanol aufgenommen und mit methanolischer Kalilauge neutralisiert. Der ausgefallene Süßstoff wurde abgesaugt und getrocknet.

2,2 g = 22%.

Vergleichsbeispiel

35,42 g (250 mMol) $P_2O_5$ wurden in 250 ml $CH_2Cl_2$ vorgelegt. Bei −25°C wurden innerhalb von 60 Minuten 62,5 Ml Triethylammonium-(acetoacetamid-N-sulfonat)-Lösung in $CH_2Cl_2$ mit einem Sulfonatgehalt von 0,05 Mol hinzugetropft. Nach weiteren 60 Minuten bei −25°C wurde wie in Beispiel B-1 aufgearbeitet. Im Reaktionsprodukt konnte dünnschichtchromatographisch kein 6-Methyl-3,4-dihydro-1,2,3-oxathiazin-4-on-2,2-dioxid bzw. dessen Kaliumsalz nachgewiesen werden.

**Patentansprüche**

1. Verfahren zur Herstellung von 6-Methyl-3,4-dihydro-1,2,3-oxathiazin-4-on-2,2-dioxid und dessen nicht-toxischen Salzen durch

a) Umsetzung eines Amidosulfonsäurederivates mit einer mindestens äquimolaren Menge des Acetoacetylierungsmittels in einem inerten organischen Lösungsmittel gegebenenfalls in Gegenwart eines Amins oder Phosphins als Katalysator zu einem Acetoacetamidderivat und

b) Ringschluß des Acetoacetamidderivats

dadurch gekennzeichnet, daß man

in Stufe a) als Amidosulfonsäurederivat ein in dem eingesetzten inerten organischen Lösungsmittel zumindest teilweise lösliches Salz der Amidosulfonsäure und als Acetoacetylierungsmittel Diketen oder Acetoacetylchlorid verwendet, daß man die Umsetzung bei Temperaturen zwischen −30 und +50°C durchführt, daß man das in dieser Stufe gebildete Acetoacetamid-N-sulfonat

in Stufe b) durch die Einwirkung der mindestens äquimolaren Menge von $SO_3$, gegebenenfalls in einem inerten anorganischen oder organischen Lösungsmittel, bei Temperaturen zwischen −70 und +175°C zum Ring des 6-Methyl-3,4-dihydro-1,2,3-oxathiazin-4-on-2,2-dioxids schließt

und daß man das hier in der Säureform anfallende Produkt dann gegebenenfalls noch in einer Stufe c) mit einer Base neutralisiert.

2. Verfahren nach Anspruch 1, dadurch gekennzeichnet, daß man in Stufe a) als Acetoacetylierungsmittel Diketen verwendet.

3. Verfahren nach Anspruch 1 oder 2, dadurch gekennzeichnet, daß man in Stufe a) das Acetoacetylierungsmittel im Überschuß bis zu 30 Mol-%, vorzugsweise nur bis zu 10 Mol-%, einsetzt.

4. Verfahren nach einem oder mehreren der Ansprüche 1 bis 3, dadurch gekennzeichnet, daß man in Stufe a) als inertes organisches Lösungsmittel ein Lösungsmittel aus der folgenden Reihe, alleine oder in Mischung, einsetzt:

halogenierrte aliphatische Kohlenwasserstoffe, vorzugsweise mit bis zu 4 C-Atomen,
aliphatische Ketone, vorzugsweise mit 3 bis 6 C-Atomen,
aliphatische Ether, vorzugsweise cyclische Ether mit 4 bis 5 C-Atomen,
niedere aliphatische Carbonsäuren, vorzugsweise mit 2 bis 6 C-Atomen,
niedere aliphatische Nitrile, vorzugsweise Acetonitril, N-Alkyl-substituierte Amide der Kohlensäure und niedere aliphatischen Carbonsäuren, vorzugsweise Amide mit insgesamt bis zu 5 C-Atomen,
aliphatische Sulfoxide, vorzugsweise Dimethylsulfoxid, und
aliphatische Sulfone, vorzugsweise Sulfolan.

5. Verfahren nach einem oder mehreren der Ansprüche 1 bis 4, dadurch gekennzeichnet, daß man in Stufe a) als inertes organisches Lösungsmittel Methylenchlorid, 1,2-Dichlorethan, Aceton, Eisessig und/oder Dimethylformamid, insbesondere Methylenchlorid, verwendet.

6. Verfahren nach einem oder mehreren der Ansprüche 1 bis 5, dadurch gekennzeichnet, daß man in Stufe a) als Amin- oder Phosphin-Katalysatoren nukleophile tertiäre Amine und Phosphine — vorzugsweise nur Amine — mit bis zu 20 — vorzugsweise nur bis zu 10 — C-Atomen pro N- oder P-Atom, insbesondere Triethylamin, verwendet.

7. Verfahren nach einem oder mehreren der Ansprüche 1 bis 6, dadurch gekennzeichnet, daß man in Stufe a) als in dem inerten organischen Lösungsmittel zumindest teilweise lösliche Salze der Amidosulfonsäure das Lithium-, $NH_4$- sowie die primären, sekundären, tertiären und/oder quartären Ammoniumsalze der Amidosulfonsäure verwendet.

8. Verfahren nach einem oder mehreren der Ansprüche 1 bis 7, dadurch gekennzeichnet, daß man Stufe a) bei Temperaturen zwischen 0 und +25°C, durchführt.

9. Verfahren nach einem oder mehreren der Ansprüche 1 bis 8, dadurch gekennzeichnet, daß man in Stufe b) das $SO_3$ in einem bis zu 20-fachen, vorzugsweise einem 3- bis 10-fachen, insbesondere einem 4- bis 7-fachen molaren Überschuß, bezogen auf das Acetoacetamid-N-sulfonat, einsetzt.

10. Verfahren nach einem oder mehreren der Ansprüche 1 bis 9, dadurch gekennzeichnet, daß man in Stufe b) als inertes anorganisches Lösungsmittel flüssiges $SO_2$ und als inertes organisches Lösungsmittel mindestens ein Lösungsmittel aus der folgenden Reihe verwendet:

halogenierte aliphatische Kohlenwasserstoffe, vorzugsweise mit bis zu 4 C-Atomen,
Kohlensäureester niederer Alkohole, vorzugsweise Kohlensäuremethyl- und -ethylester,
niedere Nitroalkane, vorzugsweise mit bis zu 4 C-Atomen,
Collidin und
Sulfolan.

11. Verfahren nach einem oder mehreren der Ansprüche 1 bis 10, dadurch gekennzeichnet, daß man sowohl in Stufe a) als auch in Stufe b) das gleiche inerte Lösungsmittel, vorzugsweise einen halogenierten aliphatischen Kohlenwasserstoff, insbesondere Methylenchlorid, verwendet und daß man die in Stufe a) erhaltene Lösung ohne Isolierung des Acetoacetamid-N-sulfonats der Ringschlußreaktion gemäß Stufe b) zuführt.

12. Verfahren nach einem oder mehreren der Ansprüche 1 bis 11, dadurch gekennzeichnet, daß man Stufe b) bei Temperaturen zwischen −40 und +10°C, durchführt.

13. Verfahren nach einem oder mehreren der Ansprüche 1 bis 12, dadurch gekennzeichnet, daß man in Stufe c) als Base eine K-Base verwendet.

14. Verfahren zur Herstellung von Acetoacetamid-N-sulfonsäure(salzen),

dadurch gekennzeichnet, daß man in einem inerten organischen Lösungsmittel

ein darin zumindest teilweise lösliches Salz der Amidosulfonsäure

mit der mindestens äquimolaren Menge an Diketen oder Acetoacetylchlorid als Acetoacetylierungsmittel, gegebenenfalls in Gegenwart eines Amin- oder Phosphin-Katalysators bei Temperaturen zwischen −30 und +50°C umsetzt

und gegebenenfalls aus dem dabei gebildeten Acetoacetamid-N-sulfonat durch Zusatz einer starken Säure die Acetoacetamid-N-sulfonsäure in Freiheit setzt.

15. Verfahren nach Anspruch 14, dadurch gekennzeichnet, daß man als Acetoacetylierungsmittel Diketen verwendet.

16. Verfahren nach Anspruch 14 oder Anspruch 15, dadurch gekennzeichnet, daß man das Acetoacetylierungsmittel im Überschuß bis zu 30 Mol-%, vorzugsweise nur bis zu 10 Mol-% einsetzt.

17. Verfahren nach einem oder mehreren der Ansprüche 14 bis 16, dadurch gekennzeichnet, daß man als inertes organisches Lösungsmittel ein Lösungsmittel aus der folgenden Reihe, alleine oder in Mischung, einsetzt:

halogenierte aliphatische Kohlenwasserstoffe, vorzugsweise mit bis zu 4 C-Atomen,
aliphatische Ketone, vorzugsweise mit 3 bis 6 C-Atomen,
aliphatische Ether, vorzugsweise cyclische Ether mit 4 bis 5 C-Atomen,
niedere aliphatische Carbonsäuren, vorzugsweise mit 2 bis 6 C-Atomen,
niedere aliphatische Nitrile, vorzugsweise Acetonitril,
N-Alkyl-substituierte Amide der Kohlensäure und niederen aliphatischen Carbonsäuren, vorzugsweise Amide mit insgesamt bis zu 5 C-Atomen,
aliphatische Sulfoxide, vorzugsweise Dimethylsulfoxid, und
aliphatische Sulfone, vorzugsweise Sulfolan.

18. Verfahren nach einem oder mehreren der Ansprüche 14 bis 17, dadurch gekennzeichnet, daß man als inertes organisches Lösungsmittel Methylenchlorid, 1,2-Dichlorethan, Aceton, Eisessig und/oder Dimethylformamid, insbesondere Methylenchlorid, verwendet.

19. Verfahren nach einem oder mehreren der Ansprüche 14 bis 18, dadurch gekennzeichnet, daß man als Amin- oder Phosphin-Katalysator nukleophile tertiäre Amine und Phosphine — vorzugsweise nur Amine — mit bis zu 20 — vorzugsweise nur bis zu 10 — C-Atome pro N- oder P-Atom, insbesondere Triethylamin, verwendet.

20. Verfahren nach einem oder mehreren der Ansprüche 14 bis 19, dadurch gekennzeichnet, daß man als in dem inerten organischen Lösungsmittel wenigstens teilweise löslichen Salz der Aminosulfonsäure das Lithium-, $NH_4$- sowie die primären, sekundären, tertiären und/oder quartären Ammoniumsalze der Amidosulfonsäure verwendet.

21. Verfahren nach einem oder mehreren der Ansprüche 14 bis 20, dadurch gekennzeichnet, daß man die Umsetzung bei einer Temperatur zwischen 0 und +25°C, durchführt.

22. Lithium- und Ammoniumsalze der Acetoacetamid-N-sulfonsäure der Formel

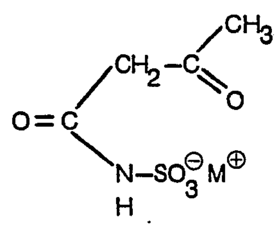

worin $M^{\oplus} = Li^{\oplus}$ oder

$\overset{\oplus}{N}R^1R^2R^3R^4$

mit $R^1$, $R^2$, $R^3$, $R^4$ = unabhängig voneinander H oder organische Reste, vorzugsweise = H, $C_1$—$C_8$-Alkyl, $C_6$—$C_{10}$-Cycloalkyl, -Aryl und/oder -Aralkyl.

23. Verfahren zur Herstellung von 6-Methyl-3,4-dihydro-1,2,3-oxathiazin-4-on-2,2-dioxid und dessen nicht toxischen Salzen durch Ringschluß eines Acetoacetamidderivats,

dadurch gekennzeichnet, daß man als Acetoacetamidderivat Acetoacetamid-N-sulfonsäure oder deren Salze verwendet und

daß man den Ringschluß durch die Einwirkung der mindestens äquimolaren Menge $SO_3$,

gegebenenfalls in einem inerten anorganischen oder organischen Lösungsmittel, bei Temperaturen zwischen −70 und +175°C durchführt,

und daß man das hier in der Säureform anfallende 6-Methyl-3,4-dihydro-1,2,3-oxathiazin-4-on-2,2-dioxid dann gegebenenfalls noch mit einer Base neutralisiert.

24. Verfahren nach Anspruch 23, dadurch gekennzeichnet, daß man das $SO_3$ in einem bis zu 20-fachen, vorzugsweise einem 3- bis 10-fachen, insbesondere einem 4- bis 7-fachen molaren Überschuß, bezogen auf die Acetoacetamid-N-sulfonsäure(salze), einsetzt.

25. Verfahren nach Anspruch 23 oder 24, dadurch gekennzeichnet, daß man als inertes anorganisches Lösungsmittel flüssiges $SO_2$

und als inertes organisches Lösungsmittel mindestens ein Lösungsmittel aus der folgenden Reihe verwendet:

halogenierte aliphatische Kohlenwasserstoffe, vorzugsweise mit bis zu 4 C-Atomen,
Kohlensäureester niederer Alkohole, vorzugsweise Kohlensäuremethyl- und -ethylester,
niedere Nitroalkane, vorzugsweise mit bis zu 4 C-Atomen,
Collidin und
Sulfolan.

26. Verfahren nach einem oder mehreren der Ansprüche 23 bis 25, dadurch gekennzeichnet, daß man die Ringschlußreaktion bei Temperaturen zwischen −40 und +10°C durchführt.

27. Verfahren nach einem oder mehreren der Ansprüche 23 bis 26, dadurch gekennzeichnet, daß man das in der Säureform anfallende 6-Methyl-3,4-dihydro-1,2,3-oxathiazin-4-on-2,2-dioxid aus dem schwefelsauren Reaktionsmedium mit einem halogenierten Lösungsmittel oder einem Ester der Kohlensäure oder einer organischen Carbonsäure extrahiert und gegebenenfalls mit einer Base die mitgerissene Schwefelsäure neutralisiert.

**Revendications**

1. Procédé de préparation du méthyl-6 dihydro-3,4 oxathiazine-1,2,3 one-4 dioxyde-2,2 et de ses sels non toxiques par:

a) réaction d'un dérivé de l'acide aminosulfonique avec une quantité au moins équimolaire d'un agent d'acétoacétylation dans un solvant organique inerte, éventuellement en présence d'une amine ou d'une phosphine comme catalyseur, pour obtenir un dérivé de l'acétoacétamide, et

b) cyclisation du dérivé de l'acétoacétamide,

procédé caractérisé en ce que:

— dans l'étape a) on utilise, comme dérivé de l'acide aminosulfonique, un sel de l'acide aminosulfonique soluble au moins partiellement dans le solvant organique inerte utilisé et, comme agent d'acétoacétylation, le dicétène ou le chlorure d'acétoacétyle, et on effectue la réaction à une température comprise entre −30 et +50°C,

— dans l'étape b) on traite l'acétoacétamide-N-sulfonate formé à l'étape précédente, par une quantité de $SO_3$ au moins équimolaire, éventuellement dans un solvant inerte organique ou non-organique, à une température comprise entre −70 et +175°C, pour créer le cycle du dioxyde-2,2 de méthyl-6 dihydro-3,4 oxathiazine-1,2,3 one-4 et,

— dans une eétape c) on neutralise ensuite éventuellement par une base le produit obtenu présentement sous la forme acide.

2. Procédé selon la revendication 1 caractérisé en ce que, dans l'étape a) on utilise le dicétène comme agent d'acétoacétylation.

3. Procédé selon l'une des revendications 1 et 2, caractérisé en ce que, dans l'étape a), on utilise l'agent d'acétoacetylation en un excès pouvant aller jusqu'à 30% en moles, de préference en un excès pouvant aller seulement jusqu'à 10% en moles.

4. Procédé selon l'une quelconque des revendications 1 à 3, caractérisé en ce que, dans l'étape a), on utilise comme solvant organique inerte un solvant, seul ou en mélange, pris dans l'ensemble constitué par:

— des hydrocarbures aliphatiques halogénés, de préference ceux qui contiennent au plus 4 atomes de carbone,

— des cétones aliphatiques, de préference celles qui contiennent de 3 à 6 atomes de carbone,

— des éthers aliphatiques, de préference des éthers cycliques contenant 4 ou 5 atomes de carbone,

— des acides carboxyliques aliphatiques inférieurs, de préférence ceux qui contiennent de 2 à 6 atomes de carbone,

— des nitriles aliphatiques inférieurs, de préference l'acétonitrile;

— des N-alkyl-amides de l'acide carbonique et d'acides carboxyliques aliphatiques inférieurs, de préférence des amides ne contenant au total pas plus de 5 atomes de carbone,

— des sulfoxydes aliphatiques, de préférence le diméthyl-sulfoxyde,

et

— des sulfones aliphatiques, de préférence le sulfolan.

5. Procédé selon l'une quelconque des revendications 1 à 4, caractérisé en ce que, dans l'étape a), on utilise comme solvant organique inerte le chlorure de méthylène, le dichloro-1,2 éthane, l'acétone, l'acide acétique glacial et/ou le diméthylformamide, plus particulièrement le chlorure de méthylène.

6. Procédé selon l'une quelconque des revendications 1 à 5, caractérisé en ce que, dans l'étape a), on utilise comme amines ou phosphines devant servir de catalyseurs, des amines ou phosphines tertiaires nucléophiles — de préference seulement des amines — qui contiennent, par atome N ou P, ou plus 20 atomes de carbone, de préference au plus 10, plus particulièrement la triéthylamine.

7. Procédé selon l'une quelconque des revendications 1 à 6, caractérisé en ce que, dans l'étape a), on utilise, comme sels de l'acide aminosulfonique au moins partiellement solubles dans le solvant organique inerte, le sel de lithium, le sel de $NH_4$ ou des sels d'ammoniums primaires, secondaires, tertiaires et/ou quaternaires de l'acide aminosulfonique.

8. Procédé selon l'une quelconque des revendications 1 à 7, caractérisé en ce qu'on effectue l'étape a) à des températures comprises entre 0 et +25°C.

9. Procédé selon l'une quelconque des revendications 1 à 8, caractérisé en ce que, dans l'étape b), on utilise le $SO_3$ en un excès molaire pouvant aller jusqu'à 20 fois, de préférence de 3 à 10 fois, plus particulièrement de 4 à 7 fois, par rapport à l'acétoacétamide-N-sulfonate.

10. Procédé selon l'une quelconque des revendications 1 à 9, caractérisé en ce que, dans l'étape b), on utilise, comme solvant minéral inerte, du $SO_2$ liquide et, comme solvant organique inerte, au moins un solvant pris dans l'ensemble constitué par:

— des hydrocarbures aliphatiques halogénés, de préférence ceux qui contiennent au plus 4 atomes de carbone,

— des esters dérivant de l'acide carbonique et d'alcools inférieurs, de préférence le carbonate de méthyle et le carbonate d'éthyle,

— des nitro-alcanes inférieurs, de préférence ceux qui contiennent au plus 4 atomes de carbone,

— la collidine

et

— le sulfolan.

11. Procédé selon l'une quelconque des revendications 1 à 10, caractérisé en ce qu'on utilise dans l'étape a) et dans l'étape b) le même solvant inerte, de préférence un hydrocarbure aliphatique halogéné, plus particulièrement le chlorure de méthylène, et en ce qu'on conduit la solution obtenue à l'étape a), sans isoler l'acétoacétamide-N-sulfonate, à la réaction de cyclisation de l'étape b).

12. Procédé selon l'une quelconque des revendications 1 à 11, caractérisé en ce qu'on effectue l'étape b) à des températures comprises entre −40 et +10°C.

13. Procédé selon l'une quelconque des revendications 1 à 12, caractérisé en ce que, dans l'étape c), on utilise comme base une base potassique.

14. Procédé de préparation de l'acide acétoacétamide-N-sulfonique (ou de ses sels), procédé caractérisé en ce qu'on fait réagir, dans un solvant organique inerte, un sel de l'acide aminosulfonique au moins partiellement soluble dans ce solvant, avec une quantité au moins équimolaire de dicétène ou de chlorure d'acétoacétyle comme agent d'acétoacétylation, éventuellement en présence d'une amine ou d'une phosphine comme catalyseur, à des températures comprises entre −30 et +50°C, et éventuellement, de l'acétoacétamide-N-sulfonate ainsi formé, on libère, par addition d'un acide fort, l'acide acétoacétamide-N-sulfonique.

15. Procédé selon la revendication 14, caractérisé en ce qu'on utilise le dicétène comme agent d'acétoacétylation.

16. Procédé selon l'une des revendications 14 et 15, caractérisé en ce que l'agent d'acétoacétylation est mis en jeu en un excès pouvant aller jusqu'à 30% en moles, de préférence pouvant aller seulement jusqu'à 10% en moles.

17. Procédé selon l'une quelconque des revendications 14 à 16, caractérisé en ce qu'on utilise, comme solvant organique inerte, un solvant, seul ou en mélange, pris dans l'ensemble constitué par:

— des hydrocarbures aliphatiques halogénés, de préférence ceux qui contiennent au plus 4 atomes de carbone,

— des cétones aliphatiques, de préférence celles qui contiennent de 3 à 6 atomes de carbone,

— des éthers aliphatiques, de préférence des éthers cycliques contenant 4 ou 5 atomes de carbone,

— des acides carboxyliques aliphatiques inférieurs, de préférence ceux qui contiennent de 2 à 6 atomes de carbone,

— des nitriles aliphatiques inférieurs, de préférence l'acétonitrile;

— des N-alkyl-amides de l'acide carbonique et d'acides carboxyliques aliphatiques inférieurs, de préférence des amides ne contenant pas plus de 5 atomes de carbone au total,

— des sulfoxydes aliphatiques, de préférence le diméthyl-sulfoxyde

et

— des sulfones aliphatiques, de préférence le sulfolan.

18. Procédé selon l'une quelconque des revendications 14 à 17, caractérisé en ce qu'on utilise, comme solvant organique inerte, le chlorure de méthylène, le dichloro-1,2 éthane, l'acétone, l'acide acétique glacial et/ou le diméthylformamide, plus particulièrement le chlorure de méthylène.

19. Procédé selon l'une quelconque des revendications 14 à 18, caractérisé en ce qu'on utilise, comme amines ou phosphines devant jouer le rôle de catalyseurs, des amines ou des phosphines tertiaires nucléophiles — de préférence seulement des amines — qui, par atome N ou P, contiennent au maximum 20 atomes de carbone, de préférence au maximum 10, plus particulièrement la triéthylamine.

20. Procédé selon l'une quelconque des revendications 14 à 19, caractérisé en ce qu'on utilise, comme sels de l'acide aminosulfonique au moins partiellement solubles dans le solvant organique inerte, le sel de lithium, le sel de $NH_4$ ou des sels d'ammoniums primaires, secondaires, tertiaires et/ou quaternaires de l'acide aminosulfonique.

21. Procédé selon l'une quelconque des revendications 14 à 20, caractérisé en ce qu'on effectue la réaction à une température comprise entre 0 et +25°C.

22. Sels de lithium et d'ammoniums de l'acide acétoacétamide-N-sulfonique qui répondent à la formule suivante:

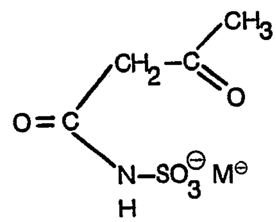

dans laquelle $M^+$ représente $Li^+$ ou représentee $N^+R^1R^2R^3R^4$, les symboles $R^1$, $R^2$, $R^3$, $R^4$ représentant chacun, indépendamment les uns des autres, H ou un radical organique, de préférence H, un radical alkyle en $C_1$—$C_8$ ou un radical cycloalkyle, aryle et/ou aralkyle en $C_6$—$C_{10}$.

23. Procédé pour préparer le méthyl-6 dihydro-3,4 oxathiazine-1,2,3 one-4 dioxyde-2,2 et ses sels non toxiques, par cyclisation d'un dérivé de l'acétoacétamide, procédé caractérisé en ce qu'on utilise, comme dérivé de l'acétoacétamide, l'acide acétoacétamide-N-sulfonique ou ses sels, on effectue la cyclisation en faisant agir une quantité au moins équimolaire de $SO_3$, éventuellement dans un solvant minéral ou organique inerte, à des températures comprises entre −70 et +175°C, et on neutralise éventuellement par une base le méthyl-6 dihydro-3,4 oxathiazine-1,2,3 one-4 dioxyde-2,2 qui a été obtenu sous sa forme acide.

24. Procédé selon la revendication 23 caractérisé en ce qu'on met en jeu le $SO_3$ en un excès molaire, par rapport à l'acide acétoacétamide-N-sulfonique (ou à ses sels), pouvant aller jusqu'à 20 fois, de préférence de 3 à 10 fois, plus particulièrement de 4 à 7 fois.

25. Procédé selon l'une des revendications 23 et 24, caractérisé en ce qu'on utilise, comme solvant minéral inerte, le $SO_2$ liquéfié et, comme solvant organique inerte, au moins un solvant pris dans l'ensemble constitué par:

— des hydrocarbures aliphatiques halogénés, de préférence ceux qui contiennent au plus 4 atomes de carbone,

— des esters dérivant de l'acide carbonique et d'alcools inférieurs, de préférence le carbonate de méthyle et le carbonate d'éthyle,

— des nitro-alcanes inférieurs, de préférence ceux qui contiennent au plus 4 atomes de carbone,

— la collidine

et

— le sulfolan.

26. Procédé selon l'une quelconque des revendications 23 à 25, caractérisé en ce qu'on effectue la réaction de cyclisation à des températures comprises entre −40 et +10°C.

27. Procédé selon l'une quelconque des revendications 23 à 26, caractérisé en ce que, du milieu réactionnel à l'acide sulfurique, on extrait par un solvant halogéné ou un ester de l'acide carbonique ou d'un acide carboxylique organique le méthyl-6 dihydro-3,4 oxathiazine-1,2,3 one-4 dioxyde-2,2 qui a été obtenu sous sa forme acide, et, éventuellement, on neutralise par une base l'acide sulfurique qui a été entraîné.

**Claims**

1. A process for the preparation of 6-methyl-3,4-dihydro-1,2,3-oxathiazin-4-one 2,2-dioxide and its non-toxic salts by

a) reaction of a sulfamic acid derivative with at least an equimolar amount of the acetoacetylating agent in an inert organic solvent, where appropriate in the presence of an amine or phosphine as catalyst, to give an acetoacetamide derivative and

b) ring closure of the acetoacetamide derivative, characterized in, in step a), the sulfamic acid derivative used being a salt of sulfamic acid which is at least partially soluble in the inert organic solvent used, and the acetoacetylating agent used being diketene or acetoacetyl chloride, and carrying out the reaction at temperatures between −30 and +50°C, and the acetoacetamide-N-sulfonate, which is formed in this step being ring-closed in step b) by the action of at least an equimolar amount of $SO_3$, where appropriate in an inert inorganic or organic solvent, at temperatures between −70 and +175°C, to form 6-methyl-3,4-dihydro-1,2,3-oxathiazin-4-one 2,2-dioxide, and the product which results from this in the acid form then being neutralized with a base, where appropriate in an additional step c).

2. The process as claimed in claim 1, wherein diketene is used as the acetoacetylating agent in step a).

3. The process as claimed in claim 1 or 2, wherein the acetoacetylating agent in step a) is used in an excess of up to 30 mol-%, preferably only up to 10 mol-%.

4. The process as claimed in one or more of claims 1 to 3, wherein the inert organic solvent used in step a) is a solvent from the following group, alone or in a mixture: halogenated aliphatic hydrocarbons, preferably having up to 4 carbon atoms,

aliphatic ketones, preferably having 3 to 6 carbon atoms,

aliphatic ethers, preferably cyclic ethers having 4 or 5 carbon atoms,

lower aliphatic carboxylic acids, preferably having 2 to 6 carbon atoms,

lower aliphatic nitriles, preferably acetonitrile,

N-alkyl-substituted amides of carbonic acid and lower aliphatic carboxylic acids, preferably amides having a total of up to 5 carbon atoms,
aliphatic sulfoxides, preferably dimethyl sulfoxide, and
aliphatic sulfones, preferably sulfolane.

5. The process as claimed in one or more of claims 1 to 4, wherein the inert organic solvent used in step a) is methylene chloride, 1,2-dichloroethane, acetone, glacial acetic acid and/or dimethylformamide, in particular methylene chloride.

6. The process as claimed in one or more of claims 1 to 5, wherein the amine or phosphine catalysts used in step a) are nucleophilic tertiary amines and phosphines — preferably only amines — having up to 20 — preferably up to only 10 — carbon atoms per N or P atom, in particular triethylamine.

7. The process as claimed in one or more of claims 1 to 6, wherein the salts of sulfamic acid which are used in step a) and are at least partially soluble in the inert organic solvent are the lithium, $NH_4$ and primary, secondary, tertiary and/or quaternary ammonium salts of sulfamic acid.

8. The process as claimed in one or more of claims 1 to 7, wherein step a) is carried out at temperatures between 0 and +25°C.

9. The process as claimed in one or more of claims 1 to 8, wherein the $SO_3$ in step b) is used in a molar excess of up to 20-fold, preferably 3- to 10-fold, in particular 4- to 7-fold, relative to the acetoacetamide-N-sulfonate.

10. The process as claimed in one or more of claims 1 to 9, wherein the inert inorganic solvent used in step b) is liquid $SO_2$, and the inert organic solvent used is at least one solvent from the following group:
halogenated aliphatic hydrocarbons, preferably having up to 4 carbon atoms,
esters of lower alcohols and carbonic acid, preferably methyl and ethyl carbonate,
lower nitroalkanes, preferably having up to 4 carbon atoms,
collidine and
sulfolane.

11. The process as claimed in one or more of claims 1 to 10, wherein the same inert solvent, preferably a halogenated aliphatic hydrocarbon, in particular methylene chloride, is used in both step a) and step b), and the solution obtained in step a) is, without isolation of the acetoacetamide-N-sulfonate, transferred to the ring closure reaction of step b).

12. The process as claimed in one or more of claims 1 to 11, wherein step b) is carried out at temperatures between −40 and +10°C.

13. The process as claimed in one or more of claims 1 to 12, wherein the base used in step c) is a K base.

14. A process for the preparation of acetoacetamide-N-sulfonic acid or salts thereof, characterized in reaction, in an inert organic solvent, of a salt of sulfamic acid, which is at least partially soluble therein, with at least the equimolar amount of diketene or acetoacetyl chloride as acetoacetylating agent, where appropriate in the presence of an amine or phosphine catalyst at temperatures between −30 and +50°C, and, where appropriate, liberation, by addition of a strong acid, of acetoacetamide-N-sulfonic acid from the acetoacetamide-N-sulfonate formed in this reaction.

15. The process as claimed in claim 14, wherein diketene is used as the acetoacetylating agent.

16. The process as claimed in claim 14 or 15, wherein the acetoacetylating agent is used in an excess of up to 30 mol-%, preferably up to only 10 mol-%.

17. The process as claimed in one or more of claims 14 to 16, wherein the inert organic solvent used is a solvent from the following group, alone or in a mixture:
halogenated aliphatic hydrocarbons, preferably having up to 4 carbon atoms,
aliphatic ketones, preferably having 3 to 6 carbon atoms,
aliphatic ethers, preferably cyclic ethers having 4 or 5 carbon atoms,
lower aliphatic carboxylic acids, preferably having 2 to 6 carbon atoms,
lower aliphatic nitriles, preferably acetonitrile, N-alkyl-substituted amides of carbonic acid and lower aliphatic carboxylic acids, preferably amides having a total of up to 5 carbon atoms,
aliphatic sulfoxides, preferably dimethyl sulfoxide, and
aliphatic sulfones, preferably sulfolane.

18. The process as claimed in one or more of claims 14 to 17, wherein the inert organic solvent used is methylene chloride, 1,2-dichloroethane, acetone, glacial acetic acid and/or dimethylformamide, in particular methylene chloride.

19. The process as claimed in one or more of claims 14 to 18, wherein the amine or phosphine catalyst used is nucleophilic tertiary amines and phosphines — preferably only amines — having up to 20 — preferably up to only 10 — carbon atoms per N or P atom, in particular triethylamine.

20. The process as claimed in one or more of claims 14 to 19, wherein the salt of sulfamic acid which is used and which is at least partially soluble in the inert organic solvent is the lithium, $NH_4$ or primary, secondary, tertiary and/or quaternary ammonium salts of sulfamic acid.

21. The process as claimed in one or more of claims 14 to 20, wherein the reaction is carried out at a temperature between 0 and +25°C.

22. Lithium and ammonium salts of acetoacetamide-N-sulfonic acid of the formula

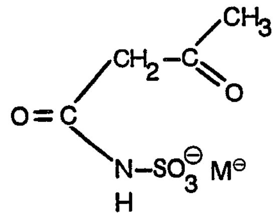

in which $M^{\oplus}$ = $Li^{\oplus}$ or
$N^+R^1R^2R^3R^4$ with
$R^1$, $R^2$, $R^3$ and $R^4$, independently of one another, = H or organic radicals, preferably = H, $C_1$—$C_8$-alkyl, $C_6$—$C_{10}$-cycloalkyl, -aryl and/or -aralkyl.

23. A process for the preparation of 6-methyl-3,4-dihydro-1,2,3-oxathiazin-4-one 2,2-dioxide and its non-toxic salts by ring closure of an acetoacetamide derivative, characterized in using as the acetoacetamide derivative acetoacetamide-N-sulfonic acid or its salts, and carrying out the ring closure by the action of at least the equimolar amount of $SO_3$, where appropriate in an inert inorganic or organic solvent, at temperatures between −70 and +175°C, and then, where appropriate, also neutralizing with a base the 6-methyl-3,4-dihydro-1,2,3-oxathiazin-4-one 2,2-dioxide which is produced in the form of the acid in this reaction.

24. The process as claimed in claim 23, wherein the $SO_3$ is used in a molar excess of up to 20-fold, preferably 3- to 10-fold, in particular 4- to 7-fold, relative to the acetoacetamide-N-sulfonic acid or salts thereof.

25. The process as claimed in claim 23 or 24, wherein the inert inorganic solvent used is liquid $SO_2$, and the inert organic solvent used is at least one solvent from the following group:
halogenated aliphatic hydrocarbons, preferably having up to 4 carbon atoms,
esters of lower alcohols and carbonic acid, preferably methyl and ethyl carbonate,
lower nitroalkanes, preferably having up to 4 carbon atoms,
collidine and
sulfolane.

26. The process as claimed in one or more of claims 23 to 25, wherein the ring closure reaction is carried out at temperatures between −40 and +10°C.

27. The process as claimed in one or more of claims 23 to 26, wherein the 6-methyl-3,4-dihydro-1,2,3-oxathiazin-4-one 2,2-dioxide, which is produced in the form of the acid, is extracted from the sulfuric acid reaction medium using a halogenated solvent or an ester of carbonic acid or of an organic carboxylic acid, and, where appropriate, the sulfuric acid which has been carried over is neutralized with a base.